# DEMANDE DE BREVET EUROPEEN

(11) **EP 2 549 259 A1**
(43) Date de publication de la demande: **23.01.2013**
(21) Numéro de dépôt: 12176716.4
(22) Date de dépôt: 17.07.2012
(51) Int. Cl.: G01N 1/12, G01W 1/14

(54) **Dispositif de collecte d'un échantillon de liquide, notamment d'eau pluviale.**

(30) Priorité: 21.07.2011 FR 1156630
(71) Demandeur: Aqua Virgo, 35760 Saint-Gregoire (FR)
(72) Inventeur: Croquison, Sébastien, 35720 Pleugueneuc (FR)
(74) Mandataire: Regimbeau

(57) **Abrégé**

L'invention concerne un dispositif de collecte d'un échantillon de liquide, notamment d'eau pluviale comprenant un récipient de collecte (2), celui-ci comprenant :
- une chambre de stockage (400) dudit échantillon, munie d'un orifice de collecte (513) et de moyens (47) d'obturation de cet orifice, ces moyens d'obturation (47) étant mobiles, de façon réversible, entre une position ouverte, dans laquelle ledit liquide peut pénétrer dans ladite chambre de stockage (400) et une position fermée, lorsque ladite chambre de stockage (400) est pleine, cette chambre de stockage étant également munie d'un orifice d'évacuation.

Ce dispositif est remarquable en ce qu'il comprend :
- des moyens (53) de détection du début et de la fin de l'événement d'écoulement de liquide ayant déclenché la collecte de l'échantillon,
- un boitier électronique (66) comprenant des moyens de datation du début et de la fin dudit événement, et des moyens de mémorisation et de stockage des données correspondantes, lesdits moyens de détection (53) activant le fonctionnement desdits moyens de datation lorsque l'événement d'écoulement de liquide commence et les désactivant lorsque l'événement cesse.

## Description

La présente invention concerne un dispositif de collecte d'un échantillon de liquide, notamment d'eaux pluviales et d'eaux usées.

Une installation classée pour la protection de l'environnement (connue sous l'acronyme "ICPE") est une installation privée ou publique dont l'exploitation peut présenter des risques pour l'environnement.

A ce titre, une installation de ce type est soumise à une réglementation stricte, notamment en ce qui concerne le bruit et les risques de pollution de l'eau et de l'air environnants et des sols.

On citera à titre d'exemple illustratif mais non limitatif : les entrepôts, les usines, les dépôts de carburants, les hôpitaux, les sociétés d'exploitation d'autoroutes.

En ce qui concerne l'eau, les exploitants d'une ICPE qui y sont astreints par la réglementation, doivent effectuer, entre autres, des prélèvements d'eau pluviale et une analyse de leurs rejets aqueux dans l'environnement, une ou plusieurs fois par an, en fonction des exigences réglementaires.

La plupart des installations précitées présentent souvent d'importantes surfaces imperméables, telles que des surfaces de stockage, des parkings, des routes, des toits.

De ce fait, ces installations possèdent généralement un bassin de rétention, qui fait office de bassin tampon, pour stocker temporairement une grosse quantité d'eau, par exemple après une pluie importante ou un orage, avant de la relâcher dans la rivière voisine ou dans le réseau d'évacuation des eaux pluviales. Ces eaux de ruissellement peuvent éventuellement être chargées en polluants, dont il convient de détecter la présence.

Les exploitants d'une ICPE qui y sont astreints par la réglementation doivent ainsi mesurer, entre autres, les valeurs de pH (potentiel d'hydrogène), DCO (demande chimique en oxygène), MES (matières en suspension) et HCT (hydrocarbures totaux) de leurs rejets aqueux.

Selon une technique connue, les exploitants d'une ICPE effectuent un prélèvement d'eau directement dans le bassin de rétention. Toutefois, dans ce cas, ce prélèvement peut correspondre à une pluie datant de plusieurs semaines ou à plusieurs épisodes pluvieux successifs. Les résultats de l'analyse de ce prélèvement ne sont donc pas représentatifs, soit parce que les valeurs mesurées ont varié entre la pluie et le moment où l'analyse est effectuée, soit parce que ces valeurs correspondent à une moyenne de plusieurs pluies successives.

De plus, avec cette technique de prélèvement, il n'est pas possible de faire une datation précise de l'échantillon d'eau pluviale collecté (c'est-à-dire une mesure de la date et de l'heure du début et de la fin d'une pluie) et il n'est pas non plus possible d'associer les valeurs mesurées à des relevés de pluviométrie des services météorologiques.

Selon une autre technique connue de l'art antérieur, les exploitants des ICPE chargent un opérateur extérieur de venir prélever les eaux pluviales par temps de pluie.

Toutefois, lorsque cet opérateur doit effectuer une collecte dans des ICPE dispersées sur une région étendue, il lui arrive de se déplacer sur un site et de ne pas pouvoir recueillir un volume de liquide suffisant, le jour où il vient effectuer cette démarche.

On connaît déjà d'après le document US 5 524 495, un dispositif de collecte d'un échantillon de liquide qui comprend une chambre de stockage d'un échantillon, munie d'un orifice de collecte susceptible d'être obturé par une bille flottante lorsque ladite chambre est pleine. Cette chambre de stockage présente également un orifice d'évacuation dont l'ouverture est commandée par une vanne.

On connaît également d'après le document GB 1093069, un pluviomètre destiné à compter le nombre de fois où un volume prédéterminé d'eau de pluie est accumulé dans un réceptacle, pendant un intervalle de temps donné. Dès que le réceptacle est plein, un compteur enregistre que le réceptacle a été rempli une fois, puis une unité de contrôle commande la vidange du réceptacle qui est alors apte à être de nouveau rempli.

Aucun de ces deux dispositifs ne permet de prélever un échantillon de liquide, de le stocker et de pouvoir lui associer la date de début et de fin de l'évènement d'écoulement de liquide ayant déclenché sa collecte.

L'invention a pour but de résoudre ces inconvénients précités de l'état de la technique et notamment de fournir un dispositif permettant de collecter un volume de liquide suffisant pour effectuer des analyses, de le stocker, de dater le prélèvement effectué et de mesurer la durée de l'évènement pluvieux ou d'écoulement des eaux usées auquel il est associé.

Un autre objectif de l'invention est de proposer un dispositif fiable qui ne puisse pas être vidé de manière accidentelle.

Un objectif supplémentaire de l'invention est de fournir un dispositif qui permette éventuellement d'alerter un opérateur en temps réel lorsque la collecte de liquide (par exemple eau de pluie) a été effectuée.

A cet effet, l'invention concerne un dispositif de collecte d'un échantillon de liquide, notamment d'eaux pluviales et/ou d'eaux usées, comprenant un récipient de collecte, celui-ci comprenant :
- une chambre de stockage dudit échantillon, munie d'un orifice de collecte et de moyens d'obturation de cet orifice, ces moyens d'obturation étant mobiles, de façon réversible, entre une position ouverte, dans laquelle ledit liquide peut pénétrer dans ladite chambre de stockage et une position fermée, dans laquelle le liquide ne peut plus y pénétrer, le passage en position fermée s'effectuant lorsque ladite chambre de stockage est pleine, cette chambre de stockage étant également munie d'un orifice d'évacuation.

Conformément à l'invention, ce dispositif de collecte comprend des moyens de détection du début et de la fin de l'événement d'écoulement de liquide ayant déclenché la collecte de l'échantillon, et un boitier électronique comprenant des moyens de datation du début et de la fin dudit événement, et des moyens de mémorisation et de stockage des données correspondantes, lesdits moyens de détection activant le fonctionnement desdits moyens de datation lorsque l'événement d'écoulement de liquide commence et les désactivant lorsque l'événement cesse.

Un tel dispositif est avantageux car il permet de récolter un échantillon dès le début de l'écoulement de liquide et de stopper cette récolte dès que le réceptacle est plein, sans que l'échantillon prélevé ne soit dilué, tout en datant le prélèvement.

Selon d'autres caractéristiques avantageuses et non limitatives de l'invention, prises seules ou en combinaison :
- le dispositif comprend une antenne et le boitier électronique comprend un module émetteur d'un signal concernant les données collectées au sujet de l'échantillon recueilli par ledit dispositif, tel qu'un émetteur radio ou GSM ou GPRS, ce module émetteur étant connecté à ladite antenne et audit boitier électronique, les données émises concernant la date et l'heure du début et de la fin de l'événement ayant déclenché la collecte et éventuellement l'identification du dispositif de collecte ;
- ladite chambre de stockage présente un orifice d'évacuation et des moyens d'obturation de celui-ci, ces moyens d'obturation étant mobiles, de façon réversible, entre une position fermée, dans laquelle le liquide stocké ne peut pas sortir de la chambre de stockage et une position ouverte, dans laquelle le liquide stocké peut en sortir, le passage d'une position à l'autre s'effectuant à l'aide d'un organe de commande desdits moyens d'obturation ;
- lesdits moyens d'obturation de l'orifice d'évacuation de la chambre de stockage comprennent une soupape présentant une tête et une tige, cette tête tendant en permanence à être maintenue en position fermée, plaquée contre ledit orifice d'évacuation, sous l'action d'un ressort de pression, et pouvant être déplacée en position ouverte, écartée de l'orifice d'évacuation, à l'aide d'un bouton poussoir agissant sur la tige de soupape, à l'encontre de la force exercée par le ressort ;
- les moyens d'obturation de l'orifice d'évacuation de la chambre de stockage comprennent une vanne ;
- lesdits moyens d'obturation de l'orifice de collecte de la chambre de stockage comprennent une soupape, dont la tête peut être déplacée sous l'action du mouvement d'un flotteur disposé à l'intérieur de la chambre de stockage, de sorte que cette tête de soupape peut être soit en position ouverte, écartée de l'orifice de collecte, tant que la chambre de stockage n'est pas pleine, soit en position fermée, dans laquelle elle obture ledit orifice de collecte, lorsque ladite chambre de stockage est pleine ou quasiment pleine ;
- le récipient de collecte comprend également une chambre intermédiaire, contigüe à ladite chambre de stockage et munie d'au moins un orifice d'entrée débouchant à l'extérieur du récipient de collecte, l'orifice de collecte de la chambre de stockage débouche à l'intérieur de cette chambre intermédiaire et les moyens de détection du début et de la fin de l'événement d'écoulement de liquide ayant déclenché la collecte de l'échantillon sont disposés dans cette chambre intermédiaire ;
- les moyens de détection comprennent d'une part un capteur actif ou un capteur à flotteur et d'autre part un interrupteur ou contact connecté auxdits moyens de datation du boitier électronique, l'interrupteur ou le contact étant agencé de façon à être activé par lesdits moyens de détection lorsque la chambre intermédiaire est pleine de liquide, et à être désactivé lorsque tel n'est pas le cas ;
- la tête de la soupape d'obturation de l'orifice de collecte est disposée à l'intérieur de ladite chambre intermédiaire ;
- le récipient de collecte est muni d'une chambre de ballast ;
- le dispositif comprend un rail de guidage destiné à être fixé verticalement et ledit récipient de collecte est muni d'un coulisseau coopérant avec ce rail de guidage, de façon que le récipient de collecte puisse coulisser le long de celui-ci ;
- le dispositif comprend un câble de manipulation relié au récipient de collecte et qui permet de déplacer celui-ci le long dudit rail de guidage, ce câble présentant une poignée à son extrémité libre, l'antenne est disposée dans cette poignée ;
- le récipient de collecte est constitué de trois éléments, à savoir un réceptacle, un bol intermédiaire et un couvercle, ces trois éléments étant aptes à être solidarisés par des moyens de fixation démontables, tels que des vis ou grenouillères, le réceptacle ayant la forme d'un tube ouvert à l'une de ses extrémités et fermé à l'autre par un fond, le bol intermédiaire ayant également la forme d'un tube ouvert à l'une de ses extrémités et fermé à l'autre par un fond, ces trois éléments étant conformés de façon que lorsqu'ils sont assemblés, le fond du bol intermédiaire obture l'embouchure du réceptacle et le couvercle obture l'embouchure du bol intermédiaire, le ou les orifice(s) d'entrée et l'orifice de collecte étant respectivement ménagés dans la paroi latérale et dans le fond du bol intermédiaire, le couvercle comprenant une enceinte étanche aux liquides pour recevoir ledit boitier électronique.

L'invention concerne également un ensemble comprenant un dispositif de collecte tel que précité et un récepteur d'un signal radio, GSM ou GPRS.

D'autres caractéristiques et avantages de l'invention apparaîtront de la description qui va maintenant en être faite, en référence aux dessins annexés, qui en représentent, à titre indicatif mais non limitatif, plusieurs modes de réalisation possibles.

Sur ces dessins :
- la figure 1 est une vue en perspective d'un premier mode de réalisation du dispositif de collecte d'un échantillon de liquide conforme à l'invention,
- la figure 2 est une vue éclatée, en perspective, des trois parties qui constituent le récipient de collecte de ce dispositif,
- la figure 3 est une vue en coupe verticale du dispositif de collecte selon la ligne III-III de la figure 1,
- la figure 4 est une vue en coupe verticale de ce même dispositif, selon la ligne IV-IV de la figure 3,
- la figure 5 est une vue éclatée, en coupe verticale, du récipient de collecte selon la ligne V-V de la figure 2, après retrait du flotteur,
- la figure 6 est une vue de face d'une variante de réalisation du récipient de collecte du dispositif de collecte conforme à l'invention, et
- la figure 7 est une vue en coupe verticale du récipient de collecte selon le plan de coupe VII - VII de la figure 6.

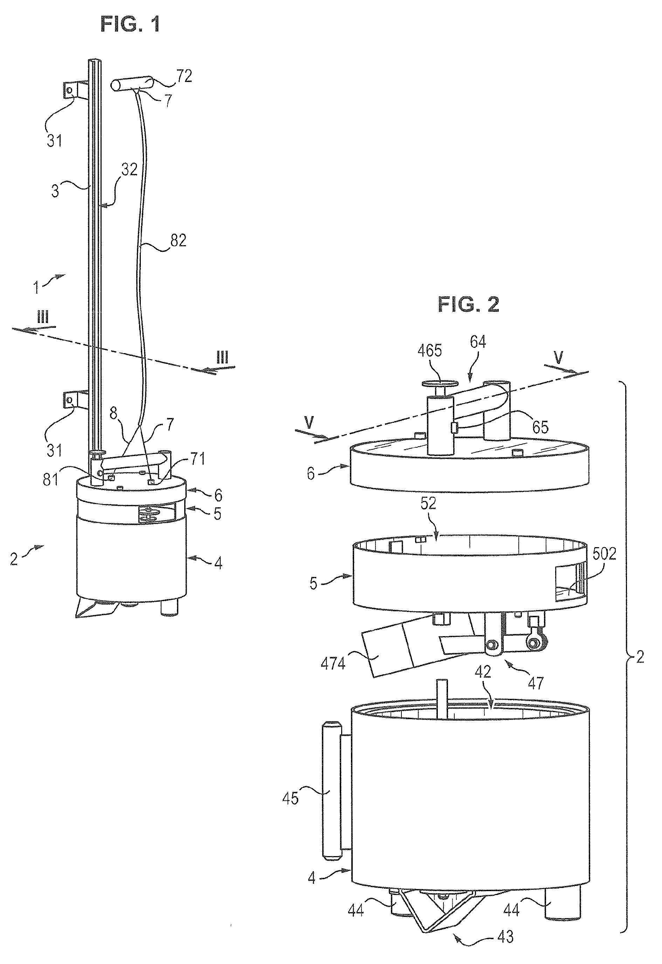

En se reportant à la figure 1, on peut voir un premier mode de réalisation d'un dispositif de collecte 1 d'un échantillon de liquide, notamment d'eau pluviale, conforme à l'invention. Ce dispositif 1 comprend un récipient de collecte 2 et un rail de guidage 3, le long duquel ce récipient peut coulisser.

Comme on peut le voir sur la figure 2, le récipient de collecte 2 se compose de trois éléments principaux, à savoir : un réceptacle 4, un bol intermédiaire 5 et un couvercle 6.

Ces trois éléments sont destinés à être assemblés par des moyens de fixation, non représentés sur les figures, tels que des vis. Ce montage est effectué de façon à pouvoir être démontable.

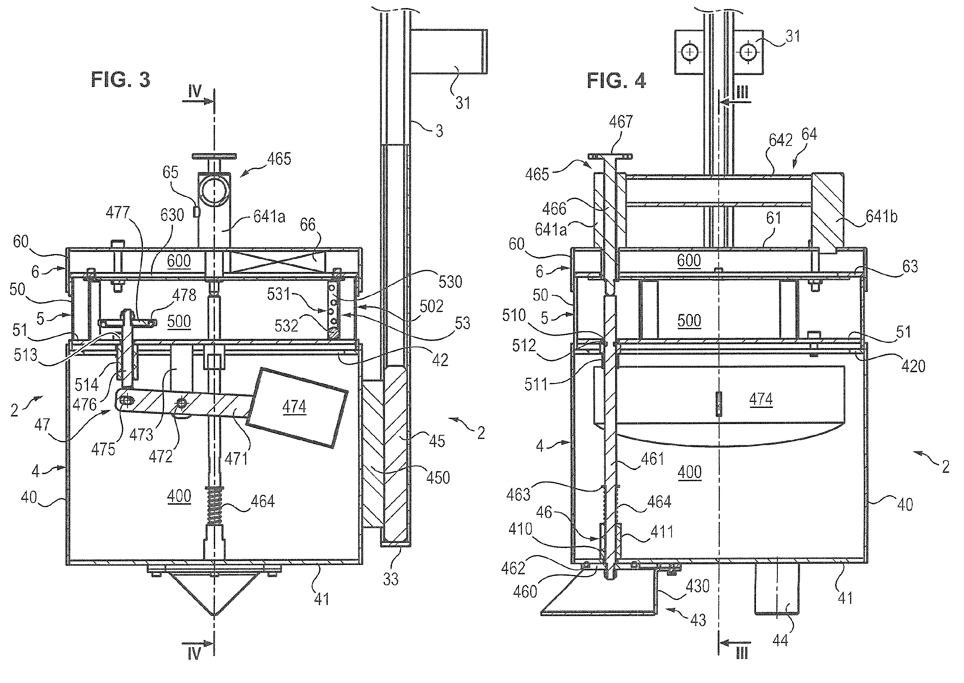

Chacun de ces éléments va maintenant être décrit plus en détail en faisant également référence aux figures 3 à 5.

Dans la suite de la description, les mentions "haut", "bas", "inférieur", "supérieur", "vertical" et "horizontal" font référence à la position normale d'utilisation du dispositif qui est celle représentée sur les figures 1 à 7.

Le réceptacle 4 présente la forme générale d'un tube, de section circulaire, bordé par une paroi latérale cylindrique 40 et dont l'extrémité inférieure est obturée par un fond circulaire 41. Le côté ouvert du réceptacle 4, dénommé embouchure, est référencé 42.

Le bol intermédiaire 5 présente une forme similaire, c'est-à-dire, la forme d'un tube de section circulaire, bordé par une paroi latérale cylindrique 50, dont l'extrémité inférieure est obturée par un fond circulaire 51 et dont l'embouchure est référencée 52.

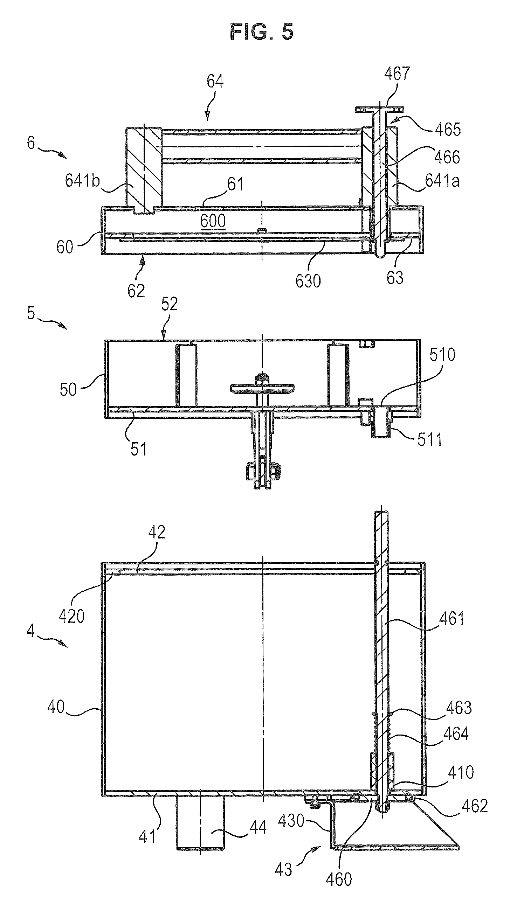

De façon avantageuse, le diamètre extérieur de la paroi latérale 50 correspond sensiblement au jeu près au diamètre intérieur de la paroi latérale 40, de sorte que le bol intermédiaire 5 peut être inséré axialement dans l'embouchure 42, et reposer sur un rebord annulaire 420 qui s'étend radialement au niveau de l'embouchure 42 (voir notamment figure 5).

Dans cette position assemblée, et comme cela apparaît mieux sur les figures 3 et 4, le fond 51 obture la partie supérieure du réceptacle 4, en délimitant ainsi à l'intérieur de celui-ci, une chambre de stockage 400 d'un échantillon de liquide.

Le couvercle 6 comprend une plaque circulaire 61, bordée par un rebord cylindrique 60 et présente une ouverture 62. En outre, une collerette annulaire 63 s'étend radialement et horizontalement, en direction de l'intérieur du couvercle, depuis le rebord 60, (voir figure 5).

Un disque 630, d'un diamètre légèrement inférieur à celui de la plaque 61, est fixé sur la collerette 63, par des moyens de fixation démontables, tels que des vis par exemple (non représentés sur les figures).

Le disque 630 s'étend parallèlement à la plaque 61, à faible distance de celle-ci et délimite avec cette plaque 61 et une partie du rebord latéral 60, une enceinte 600 étanche aux liquides.

Le diamètre intérieur de la paroi latérale 60 correspond au jeu près au diamètre extérieur de la paroi latérale 50 du bol 5, de sorte que lorsque ces deux éléments sont assemblés, le couvercle 6 vient coiffer le bol intermédiaire 5 et définit avec celui-ci une chambre intermédiaire 500 (voir figures 3 et 4).

Comme on peut le voir sur les figures 4 et 5, le fond 41 du réceptacle 4 est de préférence percé d'un orifice 410, dit "orifice d'évacuation", puisqu'il permet d'évacuer l'échantillon de liquide présent dans la chambre de stockage 400.

De façon avantageuse, un bec verseur 43 est fixé, par exemple à l'aide de vis, sous le fond 41, en regard de l'orifice d'évacuation 410.

Ce bec verseur 43 présente une section en V. Il est obturé dans sa partie arrière par une plaque verticale 430.

Dans le mode de réalisation représenté sur les figures, le réceptacle 4 est également muni de deux pieds 44, solidaires du fond 41.

De façon avantageuse, le bec verseur 43 présente une hauteur identique aux pieds 44, de sorte qu'il constitue un troisième point d'appui complémentaire qui garantit l'horizontalité du fond 41 lorsque le récipient de collecte 2 repose sur une surface horizontale.

De façon avantageuse également, le réceptacle 4 du récipient de collecte 2 est équipé d'un coulisseau 45.

Ce coulisseau 45 est constitué, par exemple, d'une portion de tige cylindrique pleine, fixée à l'extérieure de la paroi latérale 40 par une ailette 450.

Le rail de guidage 3 est destiné à être fixé verticalement le long d'un support, par exemple un mur, au moyen de plusieurs pattes de fixation 31.

Il présente la forme d'un tube creux dont le diamètre intérieur correspond sensiblement à celui du coulisseau 45. Il est en outre muni d'une fente longitudinale 32 permettant le passage de l'ailette 450.

Ce rail de guidage 3 est obturé à son extrémité inférieure par un fond 33, qui sert de butée de fin de course au coulisseau 45.

L'orifice d'évacuation 410 est obturé par des moyens qui portent la référence générale 46. Un exemple de réalisation de ces moyens est décrit ci-après.

Les moyens d'obturation 46 comprennent une soupape dont la tête 460 en forme de disque est fixée à l'extrémité d'une tige d'actionnement 461 qui lui est perpendiculaire.

La tête de soupape 460 est disposée à l'extérieur du fond 41 et à l'intérieur du bec verseur 43, en regard de l'orifice d'évacuation qu'elle obture.

Sa face interne située en regard du fond 41 présente une gorge annulaire recevant un joint d'étanchéité torique 462.

La tige d'actionnement 461 ou tige de soupape est guidée verticalement, d'une part à sa partie inférieure par un manchon 411 cylindrique, qui s'étend verticalement à l'intérieur de la chambre de stockage 400, depuis la périphérie de l'orifice 410.

La tige de soupape 461 est en outre guidée à sa partie supérieure au travers d'un orifice 510, ménagé dans le fond 51 du bol intermédiaire 5 et par un manchon de guidage tubulaire creux 511 qui s'étend verticalement à l'intérieur de la chambre de stockage 400, dans le prolongement de l'orifice 510. Les deux manchons de guidage 511 et 411 sont coaxiaux.

Un joint d'étanchéité 512 torique est disposé autour de l'extrémité supérieure de la tige de soupape 461, en regard du manchon 511, de façon à assurer l'étanchéité aux liquides entre la chambre intermédiaire 500 et la chambre de stockage 400.

La tige de soupape 461 présente dans sa partie inférieure, environ au tiers de sa hauteur, un épaulement annulaire 463. Un ressort 464, de préférence hélicoïdal, est disposé autour de la tige 461, de façon que son extrémité supérieure repose contre l'épaulement 463 et son extrémité inférieure contre le manchon de guidage 411.

Le ressort 464 tend en permanence à écarter l'épaulement 463 du manchon 411, et ce faisant, tend également en permanence à plaquer la tête de soupape 460 contre l'orifice d'évacuation 410, en obturant ce dernier.

Les moyens d'obturation 46 comprennent également un poussoir 465.

De façon avantageuse, le récipient de collecte 2 est équipé d'une poignée de manipulation. Dans le mode de réalisation illustré sur la figure 4, cette poignée, référencée 64, est solidaire du couvercle 6.

Elle se compose de deux tubes verticaux 641a, 641b reliés entre eux par un tube 642 horizontal, suffisamment espacé de la plaque 61 pour qu'un opérateur puisse passer les doigts.

De façon avantageuse, le tube 641a est creux et sert de manchon de guidage au poussoir 465, plus précisément à la tige 466 de celui-ci.

La tige 466 du poussoir est montée coaxiale à la tige 461 de soupape. Son extrémité inférieure repose contre l'extrémité supérieure de la tige 461, de sorte qu'une pression exercée sur la tête 467 du poussoir a pour effet de déplacer la tige 461 vers le bas, à l'encontre de la force du ressort 464, en écartant ainsi la tête de soupape 460 de l'orifice d'évacuation 410.

Les moyens d'obturation 46 sont donc mobiles, de façon réversible, entre une position dite "fermée", illustrée sur la figure 4, dans laquelle la tête de soupape 460 obture l'orifice d'évacuation 410, de sorte que le liquide présent dans la chambre de stockage 400 ne peut en sortir et une position dite "ouverte", dans laquelle la tête 460 est au contraire écartée de l'orifice 410, en autorisant ainsi la vidange de la chambre de stockage 400.

On notera que la disposition du poussoir 465, à proximité de la poignée 64 permet avantageusement à l'opérateur de pouvoir tenir le récipient de collecte 2 d'une seule main et, simultanément, d'actionner, par exemple avec le pouce, la tête 467 du poussoir 465.

De façon avantageuse, le poussoir 465 est équipé d'une serrure de verrouillage 65, logée dans le tube vertical 641 a (voir figure 3).

Cette serrure 65 garantit l'inviolabilité du dispositif, puisque seule une personne possédant la clé qui ouvre cette serrure pourra actionner le poussoir 465 et donc vidanger le réservoir de stockage 400.

En se reportant aux figures 2 et 3, on peut voir que la paroi latérale 50 du bol intermédiaire 5 est percée d'au moins une ouverture d'entrée 502, de préférence de deux. Cette ouverture 502 est de préférence obturée par une grille, non représentée, dont les mailles permettent de retenir les boues ou les gravillons.

Par ailleurs, le fond 51 du bol intermédiaire 5 est percé d'un orifice 513, dit "orifice de collecte", car c'est par celui-ci que le liquide pénètre dans la chambre de stockage 400.

Cet orifice de collecte 513 est muni de moyens d'obturation qui portent la référence générale 47. Un exemple de réalisation de ces moyens est décrit ci-après.

Ces moyens d'obturation comprennent une biellette 471, montée pivotante autour d'un axe 472, porté par un support 473. Le support 473 s'étend verticalement depuis le fond 51 du bol intermédiaire 5 en direction de la chambre de stockage 400.

L'axe 472 est positionné dans la partie médiane de la biellette 471.

Un flotteur 474 est fixé à l'une de ses extrémités, tandis que son autre extrémité est reliée, via un axe d'articulation 475, à la tige 476 d'une soupape d'obturation de l'orifice de collecte 513. Cette tige 476 porte une tête de soupape 477, équipée d'un joint torique d'étanchéité 478.

La tête de soupape 477 est disposée en regard de l'orifice 513 mais à l'intérieur de la chambre intermédiaire 500.

Tant que la chambre de stockage 400 est vide, le flotteur 474 tend, sous l'action de son propre poids, à maintenir en position haute l'extrémité opposée de la biellette 471 qui supporte la tige de soupape 476, en écartant de ce fait la tête de soupape 477 de l'orifice de collecte 513. Cette position est représentée sur la figure 3. Dans cette position, le liquide peut pénétrer dans la chambre de stockage 400 via l'orifice 513.

Lorsque cette chambre 400 se remplit de liquide et que ce dernier parvient au niveau du flotteur 474, le flotteur tend alors à remonter, en provoquant le pivotement de la biellette 471 autour de l'axe 472.

Ce faisant, la tête de soupape 477 descend en regard de l'orifice 513 jusqu'à obturer ce dernier. Lorsqu'un opérateur effectue la vidange de la chambre de stockage 400, le flotteur 474 revient dans sa position de repos initiale.

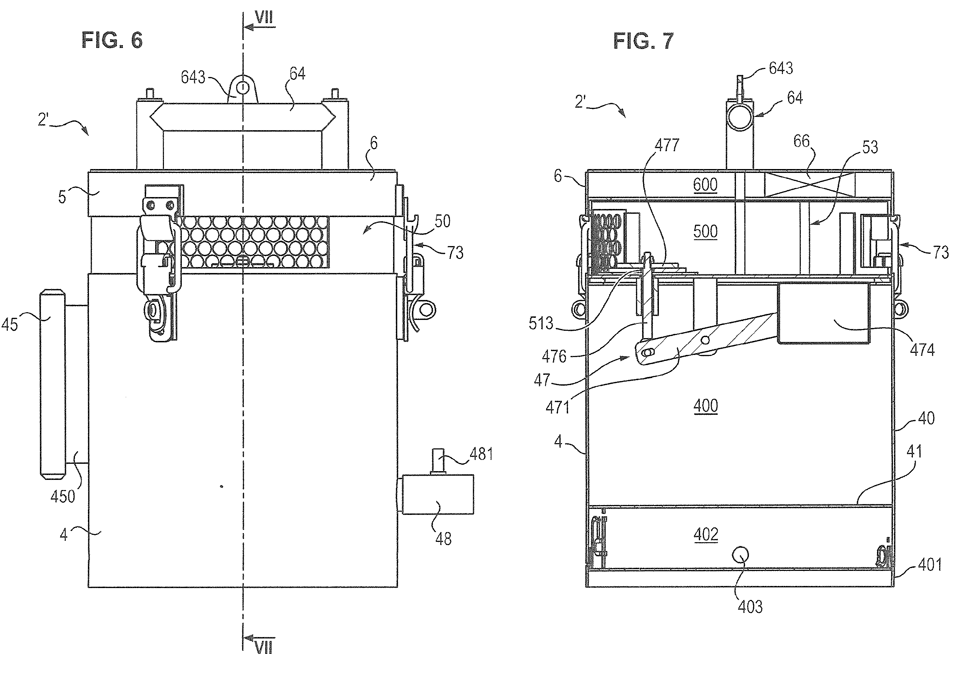

De préférence, le récipient de collecte 2 comprend également un boitier électronique 66, représenté de façon schématique uniquement sur les figures 3 et 7, et disposé à l'intérieur de l'enceinte étanche 600 du couvercle 6.

Ce boitier électronique 66 comprend des moyens de datation du début et de la fin de l'évènement ayant entraîné la collecte de l'échantillon, et des moyens de stockage et de mémorisation des données correspondantes. Ces moyens peuvent être un microcontrôleur. A titre purement illustratif, on pourra utiliser un microcontrôleur de la famille PIC, par exemple 16F8HA commercialisé par la société Microship ou un microcontrôleur de la famille AT, par exemple Atmega 328, commercialisé par la société ATMEL.

Ce boitier 66 inclut également une source d'énergie, par exemple une pile ou une batterie de type lithium, pour garantir une autonomie importante.

Par "datation", on entend la détermination de la date et de l'heure du début et de la fin de l'évènement ayant entraîné la collecte du liquide.

Par "évènement", on entend un écoulement de liquide, par exemple une pluie ou un déversement d'eaux usées.

On notera que la durée de l'évènement ne coïncide pas forcément avec la durée du prélèvement. Ainsi, par exemple, il peut commencer à pleuvoir à dix heures et cesser à douze heures, la durée de l'évènement sera alors de deux heures, tandis que le récipient de collecte sera rapidement plein, par exemple vers dix heures cinq, la durée du prélèvement sera de cinq minutes.

Ce boitier électronique 66 est logé à l'intérieur de l'enceinte 600 hermétique où il est protégé de l'humidité. Il est possible d'y accéder en démontant le disque 630.

Le récipient 2 est également équipé de moyens 53 de détection du début et de la fin de l'évènement ayant entraîné la collecte de l'échantillon. Ces moyens 53 permettent de détecter un niveau de liquide et sont positionnés par exemple dans la chambre intermédiaire 500. Ils peuvent être par exemple du type "capteur actif" ou du type "à flotteur".

Un exemple de capteur à flotteur 53 est représenté de façon schématique sur la figure 3.

Il est constitué d'un tube 530, percé d'une ou plusieurs lumières 531 et d'un organe flottant 532 (par exemple une petite bille), logé à l'intérieur du tube 530. Ce dernier est disposé verticalement à l'intérieur de la chambre intermédiaire 500.

L'organe flottant 532 est réalisé dans un matériau qui flotte à la surface du liquide collecté dans le récipient 2.

L'orifice de collecte 513 étant partiellement obturé par la tige de soupape 476, l'aire de cet orifice disponible pour le passage du liquide est relativement faible. De ce fait, lorsque le liquide pénètre dans la chambre intermédiaire 500, via l'ouverture d'entrée 502, il s'écoule au travers de l'orifice 513 en direction de la chambre de stockage 400, mais avec un débit relativement faible. Le niveau de liquide monte donc rapidement à l'intérieur de la chambre intermédiaire 500 ainsi que dans le tube 530, via les lumières 531. L'organe flottant 532 qui flotte se déplace alors vers le haut du tube 530 et vient au contact d'un interrupteur (ou contact) non représenté sur les figures et positionné au niveau du disque 630, cet interrupteur ou contact étant lui-même relié aux moyens de datation du boitier électronique 66 précité.

Ainsi, dès que le liquide pénètre dans le récipient de collecte 2, les moyens de datation du boitier électronique 66 sont activés et peuvent dater le début de l'évènement ayant entraîné la collecte de liquide, par exemple le début d'un épisode pluvieux. Inversement, lorsque le niveau de liquide redescend, l'organe flottant 532 descend également et cesse d'agir sur l'interrupteur ou le contact. Les moyens de datation du boitier électronique 66 peuvent ainsi dater la fin de l'évènement ayant entraîné la collecte de liquide, par exemple la fin de la pluie.

Un capteur actif (non représenté sur les figures) comprend par exemple deux électrodes reliées au boitier électronique 66. La montée de liquide à l'intérieur de la chambre 500 jusqu'au niveau des électrodes assure le contact électrique entre celles-ci, grâce à la conductivité du liquide, donc la fermeture du circuit électrique, et donc l'activation de l'interrupteur des moyens de datation du boitier 66 qui enregistrent le début de l'évènement ayant entraîné la collecte de liquide. Inversement, lorsque le niveau de l'eau redescend, le contact électrique entre les électrodes cesse et les moyens de datation du boitier électronique 66 enregistrent la fin de l'évènement ayant entraîné la collecte de liquide.

De façon avantageuse, le dispositif de collecte conforme à l'invention comprend également un câble 7 de manipulation. Comme on peut le voir sur la figure 1, ce câble 7 est relié à son extrémité inférieure au couvercle 6, par exemple via un système d'anneau et de mousqueton 71 et présente, à son extrémité supérieure, une poignée 72.

De façon avantageuse, le boitier électronique 66 peut également comprendre un module émetteur d'un signal radio ou GSM ou GPRS, qui permet d'envoyer un signal indiquant qu'un échantillon a été collecté dans le récipient de collecte 2. Ce module est relié à une antenne via un câble électrique 8.

Cette antenne n'est pas représentée sur les figures. Elle est avantageusement logée à l'intérieur de la poignée 72.

Le câble électrique 8 est à son tour connecté au boitier électronique 66 par une prise électrique étanche 81, par exemple un connecteur électrique tel que ceux commercialisés par la société Amphénol. Un tel connecteur électrique 81 est étanche aux liquides.

De façon avantageuse, le câble 7 et le fil électrique 8 sont logés à l'intérieur d'une gaine commune 82.

Le signal envoyé par le module émetteur peut être une onde radio (transmission sans fil) ou utiliser la technologie "wifi" ou les fréquences téléphoniques d'un téléphone mobile, tel que ceux répondant par exemple aux normes GSM ou GPRS. Ce signal permet d'envoyer à l'opérateur les informations suivantes : dates et heures du début et de la fin de l'évènement pluvieux (ou d'arrivée de liquide), et donc durée de l'évènement et éventuellement identification du dispositif de collecte émetteur (par exemple par un code ou par sa localisation géographique en coordonnées GPS). Le boitier 66 inclut alors un dispositif GPS. Ces données sont reçues sur un récepteur approprié (mobile, ordinateur, etc...) pour recevoir le signal émis par l'émetteur.

A titre d'exemple, on pourra utiliser comme module GPRS, le module TM2 (TELTONIKA) distribué par la société LEXTRONIC ou le module G20-G30 (MOTOROLA) distribué par la société FBI-OPTILAS.

A titre d'exemple purement illustratif et non limitatif, le diamètre du récipient de collecte 2 est d'environ 20 centimètres et sa hauteur totale d'environ 30 centimètres. Le volume de liquide collecté à l'intérieur de la chambre de stockage 400 est d'environ trois litres.

Les éléments constituant le récipient de collecte 2 sont avantageusement choisis parmi les matériaux inertes vis-à-vis des liquides et des produits chimiques que l'on est amené à collecter à l'intérieur. Il peut s'agir par exemple d'un métal, tel que de l'acier inoxydable ou d'une matière plastique.

Enfin, on notera que si un mode de réalisation particulier de l'invention vient d'être décrit, le récipient de collecte 2 pourrait présenter une autre forme, par exemple parallélépipédique, la poignée 64 pourrait être positionnée en un autre point du dispositif, le nombre de pieds 44 pourrait être supérieur à deux.

On notera également que l'orifice d'évacuation 410, ainsi que ses moyens d'obturation 46 et le bec verseur pourraient être supprimés, la vidange de la chambre de stockage 400 s'effectuant alors par l'embouchure 42, après démontage du bol intermédiaire 5. L'embouchure 42 joue le rôle d'orifice d'évacuation.

De même, la forme générale du récipient de collecte 2 qui est prévu pour être installé dans des regards et ou chambres de type décanteur (c'est à dire disposant dans leur fond d'une réserve d'eau) pourrait être différente, et notamment de plus faible hauteur et de plus grande largeur pour s'adapter aux différents points d'implantation du dispositif sur le terrain, par exemple à la forme d'une canalisation ou d'un regard de visite.

Une variante de réalisation du récipient de collecte, référencé 2' va maintenant être décrit en liaison avec les figure 6 et 7. Les éléments identiques au récipient 2 portent les mêmes références numériques. Seuls les éléments différents sont décrits.

La poignée est équipée d'un oeillet 643 d'accrochage du câble 7. L'assemblage des trois éléments 4, 5 et 6 est assuré par des grenouillères 73. Les pieds 44 sont remplacés par un prolongement annulaire 401 vers le bas de la paroi 40.

L'orifice d'évacuation 410 de la chambre de stockage 400 est ménagé dans la paroi latérale 40 et est obturé par une vanne 48, actionnée par une manette 481.

Enfin, le récipient de collecte 2' présente de préférence dans sa partie basse, une chambre additionnelle de ballast 402, percée d'au moins d'un orifice 403, qui se remplit d'eau lorsque le récipient est immergé et empêche ainsi sa flottaison et qui se vide lorsque le récipient est sorti de l'eau. Ceci permet de réaliser le récipient dans un matériau léger.

L'utilisation et le fonctionnement de ce dispositif de collecte vont maintenant être décrits plus en détail.

Le rail de guidage 3 est monté sur un support vertical, par exemple un mur, de préférence en aval d'un bassin de rétention ou d'un regard de visite. Ce rail reste fixé à demeure.

Lorsque l'opérateur arrive sur les lieux avec l'appareil, il accroche le câble 7 au récipient de collecte 2, 2' et branche le câble électrique 8 relié à l'antenne, (si celle-ci est présente) sur la prise 81. C'est cette action qui permet d'activer l'alimentation en énergie du boîtier électronique 66. Ainsi, pendant le transport sur son lieu d'implantation, le dispositif ne risque pas d'être activé de façon accidentelle.

Il engage ensuite le coulisseau 45 à l'intérieur du rail de guidage 3 et fait descendre le récipient de collecte 2 en le retenant à l'aide du câble 7.

On notera que le rail de guidage 3 a préalablement été positionné sur le mur de façon appropriée pour que lorsque le coulisseau 45 parvient contre la butée 33, le récipient 2 soit à une hauteur appropriée pour la collecte des échantillons (dans le cas des regards avec décantation par exemple et donc présence d'eau au fond, la partie basse est immergée jusqu'à environ 1 cm de l'orifice d'entrée 502 qui lui est hors de l'eau).

Lorsque le liquide, par exemple l'eau pluviale, pénètre dans la chambre intermédiaire 500, puis à l'intérieur de la chambre de stockage 400, il active les moyens 53 de détection du début de la collecte, conformément à ce qui a été expliqué précédemment.

La soupape 46 ou la vanne 48 étant en position fermée, le niveau du liquide monte à l'intérieur de la chambre de stockage 400 et en actionnant le flotteur 474 provoque l'obturation de l'orifice de collecte 513, dès lors que la chambre de stockage 400 est pleine ou quasiment pleine (selon la course du flotteur à l'intérieur de la chambre 400).

En outre, si le dispositif comprend un émetteur et une antenne, un signal est envoyé à l'opérateur, lui indiquant qu'une collecte de liquide a été réalisée dans le dispositif et plus précisément le début et la fin de l'évènement.

Lorsque l'opérateur revient sur les lieux, après avoir éventuellement été prévenu par l'émission du signal précité, il peut remonter le récipient de collecte 2, 2' en le hissant le long du rail de guidage 3, à l'aide du câble 7.

Après avoir récolté le liquide stocké dans le récipient 2, 2' il peut ensuite retourner celui-ci à la société chargée de sa maintenance, en vue de son démontage, son nettoyage, son dégraissage et son reconditionnement pour une nouvelle utilisation.

Le dispositif de collecte 1 conforme à l'invention permet donc de collecter un échantillon de liquide et de l'associer à une date et heure de l'évènement ayant déclenché la collecte. Ce dispositif permet plus spécifiquement de recueillir des eaux pluviales ou des eaux usées dès le début de l'écoulement, ces eaux étant potentiellement les plus chargées en polluants. Ultérieurement, les données recueillies sur cet échantillon pourront par exemple être mises en regard des données de pluviométrie fournies par les services de météorologie, (intensité et durée de la pluie).

Ce dispositif permet également de conserver l'échantillon récolté à l'abri de la lumière.

Enfin, dans le cas où ce dispositif de collecte est équipé d'un émetteur et d'une antenne, cela permet d'avertir un opérateur du fait qu'un échantillon vient d'être collecté et de rattacher cette information à un identifiant du dispositif et donc à sa position géographique.

## Revendications

1. Dispositif (1) de collecte d'un échantillon de liquide, notamment d'eaux pluviales et/ou d'eaux usées, comprenant un récipient de collecte (2, 2'), celui-ci comprenant :
- une chambre de stockage (400) dudit échantillon, munie d'un orifice de collecte (513) et de moyens (47) d'obturation de cet orifice, ces moyens d'obturation (47) étant mobiles, de façon réversible, entre une position ouverte, dans laquelle ledit liquide peut pénétrer dans ladite chambre de stockage (400) et une position fermée, dans laquelle le liquide ne peut plus y pénétrer, le passage en position fermée s'effectuant lorsque ladite chambre de stockage (400) est pleine ou quasiment pleine, cette chambre de stockage étant également munie d'un orifice d'évacuation, ce dispositif étant **caractérisé en ce qu'**il comprend :
- des moyens (53) de détection du début et de la fin de l'événement d'écoulement de liquide ayant déclenché la collecte de l'échantillon,
- un boitier électronique (66) comprenant des moyens de datation du début et de la fin dudit événement, et des moyens de mémorisation et de stockage des données correspondantes, lesdits moyens de détection (53) activant le fonctionnement desdits moyens de datation lorsque l'événement d'écoulement de liquide commence et les désactivant lorsque l'événement cesse.

2. Dispositif selon la revendication 1, **caractérisé en ce qu'**il comprend une antenne et **en ce que** le boitier électronique (66) comprend un module émetteur d'un signal concernant les données collectées au sujet de l'échantillon recueilli par ledit dispositif, tel qu'un émetteur radio ou GSM ou GPRS, ce module émetteur étant connecté à ladite antenne et audit boitier électronique (66), les données émises concernant la date et l'heure du début et de la fin de l'événement ayant déclenché la collecte et éventuellement l'identification du dispositif de collecte.

3. Dispositif selon l'une des revendications précédentes, **caractérisé en ce que** ladite chambre de stockage (400) présente un orifice d'évacuation (410) et des moyens (46, 48) d'obturation de celui-ci, ces moyens d'obturation (46, 48) étant mobiles, de façon réversible, entre une position fermée, dans laquelle le liquide stocké ne peut pas sortir de la chambre de stockage (400) et une position ouverte, dans laquelle le liquide stocké peut en sortir, le passage d'une position à l'autre s'effectuant à l'aide d'un organe de commande (465, 461, 481) desdits moyens d'obturation.

4. Dispositif selon la revendication 3, **caractérisé en ce que** lesdits moyens d'obturation (46) de l'orifice d'évacuation (410) de la chambre de stockage comprennent une soupape présentant une tête (460) et une tige (461), cette tête (460) tendant en permanence à être maintenue en position fermée, plaquée contre ledit orifice d'évacuation (410), sous l'action d'un ressort de pression (464), et pouvant être déplacée en position ouverte, écartée de l'orifice d'évacuation, à l'aide d'un bouton poussoir (465) agissant sur la tige de soupape (461), à l'encontre de la force exercée par le ressort (464).

5. Dispositif selon la revendication 3, **caractérisé en ce que** les moyens d'obturation de l'orifice d'évacuation (410) de la chambre de stockage (400) comprennent une vanne (48).

6. Dispositif selon l'une des revendications précédentes, **caractérisé en ce que** lesdits moyens d'obturation (47) de l'orifice de collecte (513) de la chambre de stockage (400) comprennent une soupape dont la tête (477) peut être déplacée sous l'action du mouvement d'un flotteur (474) disposé à l'intérieur de la chambre de stockage, de sorte que cette tête de soupape (477) peut être soit en position ouverte, écartée de l'orifice de collecte (513), tant que la chambre de stockage n'est pas pleine, soit en position fermée dans laquelle elle obture ledit orifice de collecte (513), lorsque ladite chambre de stockage (400) est pleine ou quasiment pleine.

7. Dispositif selon l'une des revendications précédentes, **caractérisé en ce que** le récipient de collecte (2, 2') comprend également une chambre intermédiaire (500), contigüe à ladite chambre de stockage (400) et munie d'au moins un orifice d'entrée (502) débouchant à l'extérieur du récipient de collecte, **en ce que** l'orifice de collecte (513) de la chambre de stockage (400) débouche à l'intérieur de cette chambre intermédiaire (500) et **en ce que** les moyens de détection (53) du début et de la fin de l'événement d'écoulement de liquide ayant déclenché la collecte de l'échantillon sont disposés dans cette chambre intermédiaire (500).

8. Dispositif selon la revendication 7, **caractérisé en ce que** les moyens de détection (53) comprennent d'une part un capteur actif ou un capteur à flotteur et d'autre part un interrupteur ou contact connecté auxdits moyens de datation du boitier électronique (66), l'interrupteur ou le contact étant agencé de façon à être activé par lesdits moyens de détection (53) lorsque la chambre intermédiaire (500) est pleine de liquide, et à être désactivé lorsque tel n'est pas le cas.

9. Dispositif selon les revendications 6 et 7, **caractérisé en ce que** la tête (477) de la soupape d'obturation de l'orifice de collecte (513) est disposée à l'intérieur de ladite chambre intermédiaire (500).

10. Dispositif selon l'une des revendications précédentes, **caractérisé en ce que** le récipient de collecte (2') est muni d'une chambre de ballast (402).

11. Dispositif selon l'une des revendications précédentes, **caractérisé en ce qu'**il comprend un rail de guidage (3) destiné à être fixé verticalement et **en ce que** ledit récipient de collecte (2, 2') est muni d'un coulisseau (45) coopérant avec ce rail de guidage (3), de façon que le récipient de collecte (2, 2') puisse coulisser le long de celui-ci.

12. Dispositif selon les revendications 2 et 11, **caractérisé en ce qu'**il comprend un câble de manipulation (7) relié au récipient de collecte (2, 2') et qui permet de déplacer celui-ci le long dudit rail de guidage (3), ce câble (7) présentant une poignée (72) à son extrémité libre, et **en ce que** l'antenne est disposée dans cette poignée.

13. Dispositif selon la revendication 7 en combinaison avec l'une quelconque des revendications précédentes, **caractérisé en ce que** le récipient de collecte (2) est constitué de trois éléments, à savoir un réceptacle (4), un bol intermédiaire (5) et un couvercle (6), ces trois éléments étant aptes à être solidarisés par des moyens de fixation démontables, tels que des vis ou grenouillères (73), le réceptacle (4) ayant la forme d'un tube ouvert à l'une de ses extrémités et fermé à l'autre par un fond (41), le bol intermédiaire (5) ayant également la forme d'un tube ouvert à l'une de ses extrémités et fermé à l'autre par un fond (51), ces trois éléments étant conformés de façon que lorsqu'ils sont assemblés, le fond (51) du bol intermédiaire (5) obture l'embouchure (42) du réceptacle (4) et le couvercle (6) obture l'embouchure (52) du bol intermédiaire (5), le ou les orifice(s) d'entrée (502) et l'orifice de collecte (513) étant respectivement ménagés dans la paroi latérale (50) et dans le fond (51) du bol intermédiaire (5), le couvercle (6) comprenant une enceinte (600) étanche aux liquides pour recevoir ledit boitier électronique (66).

14. Ensemble comprenant un dispositif de collecte d'un échantillon de liquide selon la revendication 2 en combinaison avec l'une quelconque des autres revendications précédentes et un récepteur d'un signal radio, GSM ou GPRS.
